(19) Europäisches Patentamt
European Patent Office
Office européen des brevets

(11) **EP 4 548 845 A1**

(12) **EUROPEAN PATENT APPLICATION**
published in accordance with Art. 153(4) EPC

(43) Date of publication:
**07.05.2025 Bulletin 2025/19**

(51) International Patent Classification (IPC):
**A61B 5/1455** (2006.01)   **A61B 5/0225** (2006.01)

(21) Application number: 23845624.8

(22) Date of filing: **27.07.2023**

(86) International application number:
**PCT/CN2023/109470**

(87) International publication number:
**WO 2024/022416 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.07.2022 CN 202210892732**

(71) Applicant: **Beijing Choice Electronic Technology
Co., Ltd.
Beijing 100041 (CN)**

(72) Inventors:
• **QIAN, Xiaolun
Beijing 100041 (CN)**
• **MA, Chuanlong
Beijing 100041 (CN)**
• **ZHU, Shuangshuang
Beijing 100041 (CN)**
• **PAN, Qingxi
Beijing 100041 (CN)**
• **FAN, Zhiqiang
Beijing 100041 (CN)**

(74) Representative: **Arnold & Siedsma
Bezuidenhoutseweg 57
2594 AC The Hague (NL)**

(54) **MEASUREMENT DEVICE AND METHOD, AND STORAGE MEDIUM**

(57)    A measurement device and method, and a storage medium, belonging to the technical field of medical devices. The measurement device comprises an upper housing; a lower housing; a blood oxygen collection apparatus embedded and an electrocardiogram collection apparatus which are embedded in a groove of the upper housing; and a power supply apparatus, and a circuit mainboard which are arranged between the upper housing and the lower housing, wherein the electrocardiogram collection apparatus comprises a first electrode plate and a second electrode plate; the two electrode plates are separately embedded in the groove at both ends of the upper housing; a hollow-out part is formed on any one of the electrode plates, and the blood oxygen collection apparatus is arranged at the hollow-out part. The embodiments of the present disclosure are applicable to a process of collecting blood oxygen and electrocardiogram.

FIG. 1

**Description**

The present application claims priority to a Chinese patent application No. 202210892732.0, entitled "MEASUREMENT DEVICE AND METHOD, AND STORAGE MEDIUM", filed on July 27, 2022, the entire contents of which are incorporated herein by reference.

Technical Field

Embodiments of the present disclosure relate to the technical field of medical devices, and particularly relate to a measurement device, a method, and a storage medium.

Background

Existing electrocardiogram and blood oxygen measurement requires an electrocardiogram measurement device and a blood oxygen measurement device to be detected separately, which consumes too much time of users for measurement. In addition, both the above-mentioned measurement devices have large volumes, which is inconvenient for users to record electrocardiogram and blood oxygen data anytime and anywhere. Especially when medical staff is required to intervene in the user's diagnosis and treatment process, two measurement devices are required to detect alternately, and electrocardiogram and blood oxygen data cannot be obtained at the same time, and no accurate physiological reference data can be provided for medical staff.

Summary

An object of embodiments of the present disclosure is to provide a leakage compensation circuit, a chip, and an electronic device that samples a leakage current of an output stage and inputs the sampled current back to the output stage, thereby compensating the leakage current in the output stage.

An object of the embodiments of the present disclosure is to provide measurement device and method, and a storage medium, which realize simultaneous measurement of electrocardiogram and blood oxygen by an electrocardiogram measurement apparatus integrated with a blood oxygen measurement apparatus, which reduces a volume of the measurement device.

In order to achieve the abovementioned object, a measurement device is provided in a first aspect of the embodiments of the present disclosure. The measurement device includes an upper housing and a lower housing, and a blood oxygen collection apparatus, an electrocardiogram collection apparatus and a display screen lens which are embedded in a groove of the upper housing, and a power supply apparatus, a circuit mainboard and a key which are provided between the upper housing and the lower housing; wherein the electrocardiogram collection apparatus includes a first electrode slice and a second electrode slice that are embedded in grooves at both ends of the upper housing, respectively; a hollow portion is provided at a center position of any one of the electrode slices, and the blood oxygen collection apparatus is disposed at the hollow portion.

Further, through holes are provided on the upper housing and the circuit mainboard, both the first electrode slice and the second electrode slice are provided with pins, and two pins connect the two electrode slices with the circuit mainboard through the through holes on the upper housing and the through holes on the circuit mainboard.

Further, the circuit mainboard includes a blood oxygen sensor, a display screen and a data processing module, wherein the blood oxygen sensor includes a light emitting diode and a receiving diode, the light emitting diode is configured to emit red light and infrared light, the receiving diode is configured to receive multi-channel optical signals resulting from reflecting or transmitting the red light and infrared light from human tissues, convert the multi-channel optical signals into a digital signal and transmit the digital signal to the data processing module, wherein a data signal includes red light Photoplethysmography (PPG) waveform data and infrared light PPG waveform data; the data processing module is configured to receive the digital signal and the electrocardiogram data, convert the digital signal into blood oxygen data, and obtain a blood pressure value by processing the infrared light PPG waveform data and the electrocardiogram data; and the display screen is configured to display the blood oxygen data, the electrocardiogram data and the blood pressure value through the display screen lens in the groove of the upper housing.

Further, a first aperture, a baffle and a second aperture are provided side by side in a groove of the upper housing directly below the hollow portion, wherein the first aperture is configured to transmit the red light and infrared light emitted by the light emitting diode to the blood oxygen collection apparatus; the second aperture is configured to transmit the multi-channel optical signals to the receiving diode; and the baffle is configured to isolate the light emitted by the light emitting diode from the multi-channel optical signals received by the receiving diode.

Further, the blood oxygen collection apparatus includes a light emitting lens, a reflecting lens and a light shielding pad, wherein the light shielding pad is provided with a third aperture and a fourth aperture, the third aperture is disposed above the first aperture, and the fourth aperture is disposed above the second aperture; the light emitting lens is disposed above the third aperture, and is configured to transmit red light and infrared light emitted by the light emitting diode, and filter out other light other than the red light and infrared light; the reflecting lens is disposed above the fourth aperture, and is configured to transmit the multi-channel optical signals and filter out other optical signals other than the multi-channel optical signals; and the light shielding pad is configured to block an influence of external light on transmission of the red light, the infrared light and the multi-channel optical signals.

Further, the two electrode slices are made of gold plated copper or nickel plated copper.

Further, the key is configured to start or stop acquiring the electrocardiogram data and the blood oxygen data of the user, when pressed once in control.

Further, the measurement device further includes a through hole provided on either side of the measurement device for tethering the measurement device.

Further, the power supply apparatus includes a wireless charging receiver module and a power supply module, the wireless charging receiver module is configured to receive electric energy and charge the power supply module; and the power supply module is configured to supply power to the blood oxygen sensor, the display screen and the data processing module.

Further, the data processing module includes an Analog Front End (AFE) chip, a first Microprogrammed Control Unit (MCU), a serial interface, a second MCU, a data storage module, a clock module and a communication module; wherein the AFE chip is configured to simultaneously acquire the digital signal and electrocardiogram data, and convert the digital signal into blood oxygen data; the first MCU is configured to process the infrared light PPG waveform data and the electrocardiogram data to obtain the blood pressure value; the serial interface is configured to transfer the blood oxygen data, the electrocardiogram data and the blood pressure value from the first MCU to the second MCU; the second MCU is configured to receive the blood oxygen data, the electrocardiogram data and the blood pressure value, and transmit the blood oxygen data, the electrocardiogram data and the blood pressure value to the data storage module, the display screen and the communication module; the data storage module is configured to store the blood oxygen data, the electrocardiogram data and the blood pressure value; the communication module is configured to transmit the blood oxygen data, the electrocardiogram data and the blood pressure value to a communication terminal; and the clock module is configured to provide time.

Further, obtaining the blood pressure value by processing the infrared light PPG waveform data and the electrocardiogram data includes: acquiring, when a waveform of the electrocardiogram data is stable, a pulse wave peak subsequent to the electrocardiogram wave peak in the infrared light PPG waveform data whenever the electrocardiogram wave peak is acquired, and determining the electrocardiogram wave peak and the pulse wave peak as a group of electrocardiogram and pulse wave peaks; selecting a specified time of the infrared light PPG waveform data from a duration between the time corresponding to the electrocardiogram peak and the time corresponding to the pulse wave peak in the group of electrocardiogram and pulse wave peaks; and obtaining a blood pressure value corresponding to each group of electrocardiogram and pulse wave peaks according to a first preset parameter, a second preset parameter and the specified time.

Further, the specified time includes times corresponding to a maximum value, a minimum value, a first derivative, a second derivative, or a tangent point value in the infrared light PPG waveform data, from the duration between the time corresponding to the electrocardiogram peak and the time corresponding to the pulse wave peak in the group of electrocardiogram and pulse wave peaks.

Further, the first derivative is a maximum value among differences between two adjacent pieces of data in the infrared light PPG waveform data, and the second derivative is a maximum value among differences between the two adjacent difference values.

Further, obtaining the blood pressure value corresponding to each group of electrocardiogram and pulse wave peaks according to the first preset parameter, the second preset parameter and the specified time includes: according to

$$\mathrm{BP} = \frac{a}{PAT} + b$$ or $$\mathrm{BP} = \frac{a}{PAT^2} + b$$ or BP = a · ln(PAT) + b, obtaining the blood pressure value, wherein BP is a specified time, PATa is a first preset parameter, and b is a second preset parameter.

Further, the blood pressure value includes a diastolic blood pressure and a systolic blood pressure, wherein the first preset parameter includes a first preset parameter for the diastolic blood pressure and a first preset parameter for the systolic blood pressure, and the second preset parameter includes a second preset parameter for the diastolic blood pressure and a second preset parameter for the systolic blood pressure.

Accordingly, a measurement method for the measurement device as described above is provided in a second aspect of the embodiments of the present disclosure, the measurement method includes acquiring infrared light PPG waveform data, blood oxygen data and electrocardiogram data at the same time; acquiring, when a waveform of the electrocardiogram data is stable, a pulse wave peak subsequent to the electrocardiogram wave peak in the infrared light PPG waveform data whenever the electrocardiogram wave peak is acquired, and determining the electrocardiogram wave peak and the pulse wave peak as a group of electrocardiogram and pulse wave peaks; selecting a specified time of the infrared light PPG waveform data from a duration between the time corresponding to the electrocardiogram peak and the time corresponding to the pulse wave peak in the group of electrocardiogram and pulse wave peaks; and obtaining a blood pressure value corresponding to each group of electrocardiogram and pulse wave peaks according to a first preset parameter, a second preset parameter and the specified time.

Further, the specified time includes times corresponding to a maximum value, a minimum value, a first derivative, a second derivative, or a tangent point value in the infrared light PPG waveform data, from the duration between the time corresponding to the electrocardiogram peak and the time corresponding to the pulse wave peak in the group of electrocardiogram and pulse wave peaks.

Further, the first derivative is a maximum value among differences between two adjacent pieces of data in the infrared light PPG waveform data, and the second derivative is a maximum value among differences between the two adjacent difference values.

Further, obtaining the blood pressure value corresponding to each group of electrocardiogram and pulse wave peaks according to the first preset parameter, the second preset parameter and the specified time includes: according to

$$\text{BP} = \frac{a}{PAT} + b \quad \text{or} \quad \text{BP} = \frac{a}{PAT^2} + b$$ or BP = a · ln(PAT) + b, obtaining the blood pressure value, wherein BP is the specified time, PATa is a first preset parameter, and b is a second preset parameter.

Further, the blood pressure value includes a diastolic blood pressure and a systolic blood pressure, wherein the first preset parameter includes a first preset parameter for the diastolic blood pressure and a first preset parameter for the systolic blood pressure, and the second preset parameter includes a second preset parameter for the diastolic blood pressure and a second preset parameter for the systolic blood pressure.

Accordingly, a machine-readable storage medium having instructions stored thereon for causing a machine to perform the measurement method described above is provided in a third aspect of the embodiments of the present disclosure.

According to the technical solutions, the blood oxygen measurement device and the electrocardiogram measurement device are integrated together, which reduces a volume of the measurement device, and is convenient for users to carry. In addition, the blood oxygen data and the electrocardiogram data at the same time can be acquired simultaneously by one measurement, which reduces measurement times for the users and improves a measurement efficiency of the users.

Other features and advantages in the embodiments of the present disclosure will be described in detail in the following detailed description.

Brief Description of Drawings

The accompanying drawings are intended to provide a further understanding of the embodiments of the present disclosure, and constitute a part of the specification, and together with the following detailed description, serve to explain the embodiments of the present disclosure, but are not intended to limit the embodiments of the present disclosure. In the drawings:

FIG. 1 is a schematic diagram of a structure of a measurement device according to an embodiment of the present disclosure.
FIG. 2 is a schematic top view of a measurement device according to an embodiment of the present disclosure.
FIG. 3 is an installation schematic of pins of electrode slices according to an embodiment of the present disclosure.
FIG. 4 is a schematic diagram of a structure of a circuit mainboard 17 according to an embodiment of the present disclosure.
FIG. 5 is a schematic diagram of a structure of a data processing module according to an embodiment of the present disclosure.
FIG. 6 is a schematic diagram of structures of a blood oxygen collection apparatus 13, an upper housing 11, and a blood oxygen sensor 171 according to an embodiment of the present disclosure.
FIG. 7 is a schematic diagram of an application architecture of a measurement device according to an embodiment of the present disclosure.
FIG. 8 is a flowchart of a measurement method for the measurement device described above according to an embodiment of the present disclosure.

Detailed Description

To make the objectives, technical solutions and advantages of the embodiments of the present disclosure clearer, technical solutions of the embodiments of the present disclosure will be clearly and completely described below in combination with the accompany drawings. Apparently, the described embodiments are a part of the embodiments of the present disclosure, not all of the embodiments. Based on the described embodiments of the present disclosure, all other embodiments obtained by those of skilled in the art without inventive effort are covered by the scope of protection of the present disclosure.

Unless otherwise defined, all terms (including technical and scientific terms) used herein have the same meaning as commonly understood by those of skilled in the art to which the disclosed subject matter belongs. It is further understood that terms as defined in commonly used dictionaries should be construed to have meanings consistent with their meanings in the context of the specification and related art and are not to be construed in an idealised or overly formal manner unless explicitly defined otherwise herein.

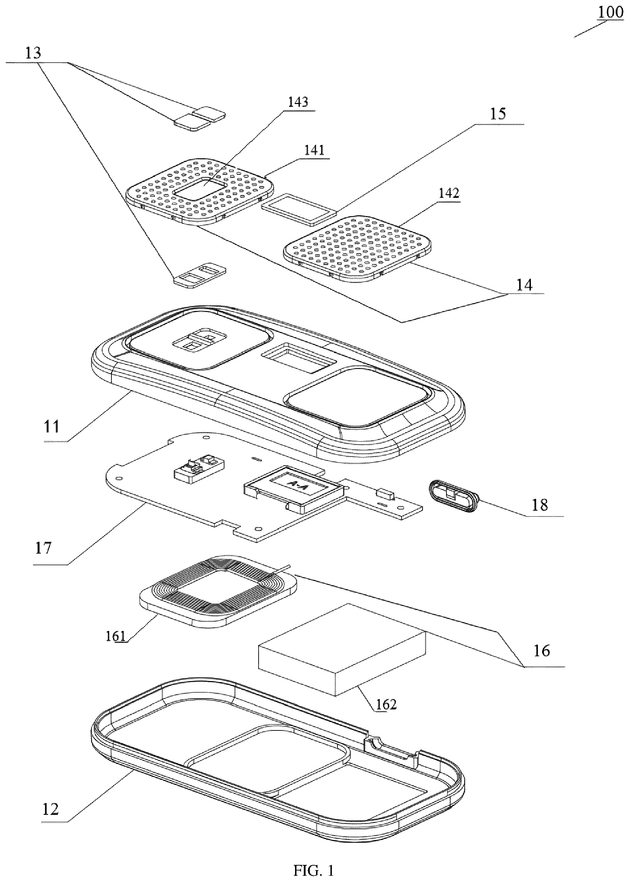

FIG. 1 is a schematic diagram of a structure of a measurement device according to an embodiment of the present disclosure. As shown in FIG. 1, the measurement device 100 includes an upper housing 11 and a lower housing 12, and a blood oxygen collection apparatus 13, an electrocardiogram collection apparatus 14 and a display screen lens 15 which are embedded in a groove of the upper housing 11, and a power supply apparatus 16, a circuit mainboard 17 and a key 18 which are provided between the upper housing 11 and the lower housing 12.

The electrocardiogram collection apparatus 14 includes a first electrode slice 141 and a second electrode slice 142 that are embedded in grooves at both ends of the upper housing, respectively. Furthermore, a hollow portion 143 is provided at a center position of any one of the electrode slices, and the blood oxygen collection apparatus 13 is disposed at the hollow portion 143.

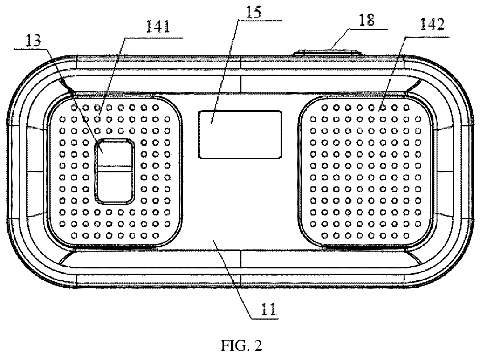

Taking the hollow portion 143 disposed at the first electrode slice 141 as an example, as shown in FIG. 2, the blood oxygen collection apparatus 13 is disposed at the center position of the first electrode slice 141. Left and right hands of a user which contact the first electrode slice 141 and the second electrode slice 142, respectively, inevitably contact the blood oxygen collection apparatus 13, so that blood oxygen data and electrocardiogram data of the user can be acquired at the same time. Combining the two kinds of measurement devices reduces a volume of the measurement devices and is convenient for users to carry. In addition, two kinds of data can be obtained at the same time with a single measurement, thus reducing the users' measurement time and improving users' measurement efficiency.

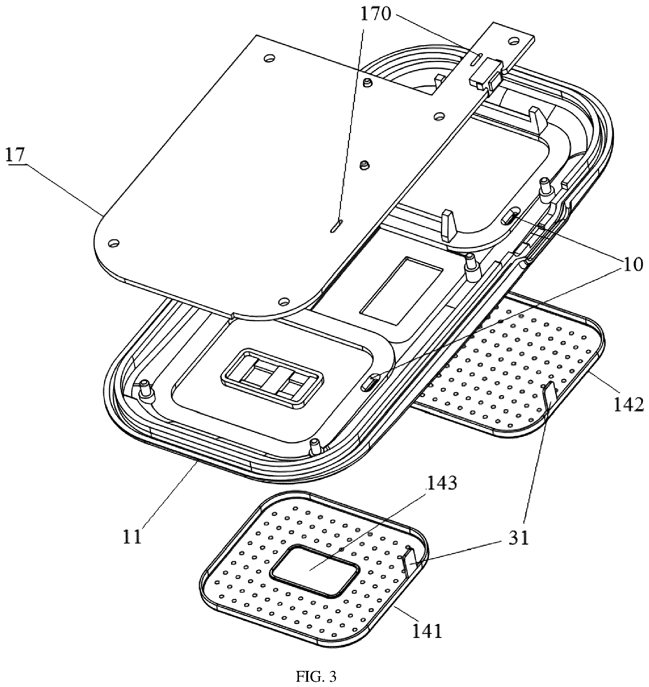

As shown in FIG. 3, a through hole 10 is provided on the upper housing 11, another through hole 170 is provided on the circuit mainboard 17, both the first electrode slice 141 and the second electrode slice 142 are provided with pins 31, and the two pins 31 connect the two electrode slices 141, 142 with the circuit mainboard 17 through the through hole 10 of the upper housing 11 and the through hole 170 on the circuit mainboard 17, so that the circuit mainboard can obtain the electrocardiogram data collected by the two electrode slices. Further, the two electrode slices are made of gold plated copper or nickel plated copper.

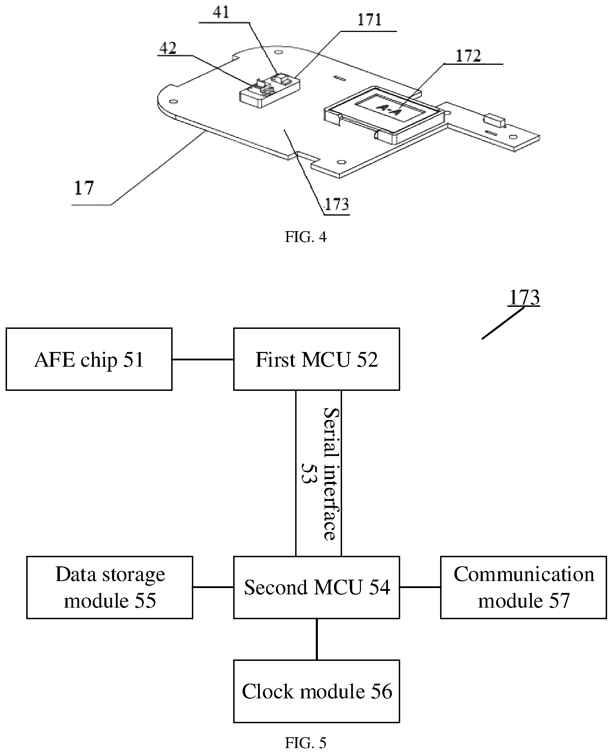

Further, as shown in FIG. 4, the circuit mainboard 17 includes a blood oxygen sensor 171, a display screen 172 and a data processing module 173.

The blood oxygen sensor 171 includes a light emitting diode 41 and a receiving diode 42. The light emitting diode 41 is configured to emit red light and infrared light. The receiving diode 42 is configured to receive multi-channel optical signals resulting from reflecting or transmitting the red light and infrared light from human tissues, convert the multi-channel optical signals into a digital signal and transmit the digital signal to the data processing module 173. The data signal includes red light PPG waveform data and infrared light PPG waveform data.

The data processing module 173 is configured to receive the digital signal and the electrocardiogram data, convert the digital signal into blood oxygen data, and obtain a blood pressure value by processing the infrared light PPG waveform data and the electrocardiogram data.

The display screen 172 is configured to display the blood oxygen data, the electrocardiogram data and the blood pressure value through the display screen lens in the groove of the upper housing. A dimension of the display screen can be determined according to a size of the measurement device, and is preferably 0.49 inches in the embodiment of the present disclosure.

As shown in FIG. 5, the data processing module 173 includes an AFE chip 51, a first MCU 52, a serial interface 53, a second MCU 54, a data storage module 55, a clock module 56 and a communication module 57.

The AFE chip 51 is configured to simultaneously collect the digital signal and electrocardiogram data, and convert the digital signal into the blood oxygen data. The first MCU 52 is configured to process the infrared light PPG waveform data and the electrocardiogram data to obtain the blood pressure value. The serial interface 53 is configured to transfer the blood oxygen data, the electrocardiogram data and the blood pressure value from the first MCU to the second MCU. The second MCU 54 is configured to receive the blood oxygen data, the electrocardiogram data and the blood pressure value, and transmit the blood oxygen data, the electrocardiogram data and the blood pressure value to the data storage module, the display screen and the communication module. The data storage module 55 is configured to store the blood oxygen data, the electrocardiogram data and the blood pressure value. The communication module 57 is configured to transmit the blood oxygen data, the electrocardiogram data and the blood pressure value to a communication terminal. The clock module 56 is configured to provide time.

A specific implementation of obtaining the blood pressure value by processing the infrared light PPG waveform data and the electrocardiogram data is as follows.

When a waveform of the electrocardiogram data is stable, a pulse wave peak subsequent to the electrocardiogram wave peak is acquired in the infrared light PPG waveform data whenever the electrocardiogram wave peak is acquired, and the electrocardiogram wave peak and the pulse wave peak are determined as a group of electrocardiogram and pulse wave peaks. That is, when the waveform of the electrocardiogram data is stable, a first electrocardiogram wave peak is acquired, a first pulse wave peak appears after the first electrocardiogram wave peak is acquired in the infrared light PPG waveform data, and the first electrocardiogram wave peak and the first pulse wave peak are determined as a first group of electrocardiogram and pulse wave peaks. Subsequently, a second electrocardiogram peak is obtained, and a second pulse wave peak appeared subsequent to the second electrocardiogram peak in the infrared light PPG waveform data is acquired. Similarly, the second electrocardiogram peak and the second pulse wave peak are determined as a second group of electrocardiogram and pulse wave peaks, and following groups of electrocardiogram and pulse wave peaks are determined by analogy, thereby obtaining a plurality of groups of electrocardiogram and pulse wave peaks. Subsequently, a specified time of the infrared light PPG waveform data is selected from a duration between the time corresponding to the electrocardiogram peak and the time corresponding to the pulse wave peak in the group of electrocardiogram and pulse wave peaks. The specified time includes times corresponding to a maximum value, a minimum value, a first derivative, a second derivative, or a tangent point value in the infrared light PPG waveform data, from the duration between the time corresponding to the electrocardiogram wave peak and the time corresponding to the pulse wave peak in the group of electrocardiogram and pulse wave peaks. In addition, the first derivative is a maximum value among differences between two adjacent pieces of data in the infrared light PPG waveform data. The second derivative is a maximum value among differences between the two adjacent difference values. That is, after calculating the first derivative, the maximum value obtained by further differentiating the two adjacent difference values among all the obtained difference values is the second derivative.

Then, according to a first preset parameter, a second preset parameter and the specified time, a blood pressure value corresponding to each group of electrocardiogram and pulse wave peaks is obtained.

In a process of calculating a Blood Pressure (BP) value according to the embodiment of the present disclosure, any one of the following three formulas may be used:

$$\mathrm{BP} = \frac{a}{PAT} + b \qquad\qquad\qquad \text{formula (1)}$$

$$\mathrm{BP} = \frac{a}{PAT^2} + b \qquad\qquad\qquad \text{formula (2)}$$

$$\mathrm{BP} = a \cdot \ln(PAT) + b \qquad\qquad\qquad \text{formula (3)}$$

where $PAT$ is the specified time, $a$ is a first preset parameter, and b is a second preset parameter.

In addition, the blood pressure value includes a diastolic blood pressure and a systolic blood pressure, for which corresponding first preset parameter and second preset parameter are also set, respectively. That is, the first preset parameter includes a first preset parameter for the diastolic blood pressure and a first preset parameter for the systolic blood pressure, and the second preset parameter includes a second preset parameter for the diastolic blood pressure and a second preset parameter for the systolic blood pressure. In an embodiment of the present disclosure, the first derivative is optimally selected for the specified time, and the formula (3) is optimally selected for calculating the blood pressure value.

In addition, prior to being displayed on the display screen of the measurement device, the obtained electrocardiogram data, the blood oxygen data and the blood pressure value can also be transmitted to the communication terminal through the communication module, for example, to a user's mobile phone for display in real time by means of Bluetooth communication.

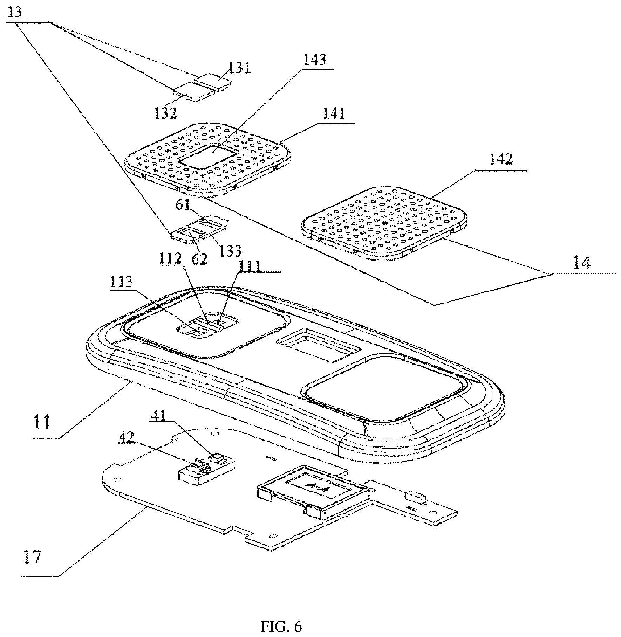

As shown in FIG. 6, a first aperture 111, a baffle 112 and a second aperture 113 are provided side by side in a groove of the upper housing 11 directly below the hollow portion 143. The first aperture 111 is configured to transmit the red light and infrared light emitted by the light emitting diode 41 to the blood oxygen collection apparatus 13. The second aperture 113 is configured to transmit the multi-channel optical signals to the receiving diode 42. The baffle 112 is configured to isolate the light emitted by the light emitting diode 41 from the multi-channel optical signals received by the receiving diode 42. In addition, as shown in FIG. 6, the blood oxygen collection apparatus 13 includes a light emitting lens 131, a reflecting lens 132 and a light shielding pad 133, wherein the light shielding pad 133 is provided with a third aperture 61 and a fourth aperture 62, the third aperture 61 is disposed above the first aperture 111, and the fourth aperture 62 is disposed above the second aperture 113. The light emitting lens 131 is disposed above the third aperture 61, and is configured to transmit red light and infrared light emitted by the light emitting diode 41, and filter out other light other than the red light and infrared light. The reflecting lens 132 is disposed above the fourth aperture 62, and is configured to transmit the multi-channel optical signals and filter out other optical signals other than the multi-channel optical signals. The light shielding pad 133 is configured to block an influence of external light on transmission of the red light, the infrared light and the multi-channel optical signals.

In addition, in order to further increase a contact area between a finger and the lens, the light emitting lens and the reflecting lens are lenses with uniform convex surfaces, thereby further increasing the quantity of blood oxygen signals collected from the finger.

In addition, in the measurement device shown in FIG. 1, the key 18 is configured to start or stop acquiring the electrocardiogram data and the blood oxygen data of the user, when pressed once in control. That is, a measurement device, in the turned-off state, is turned on by pressing the key 18, and a measurement device, in the turned-on state, is turned off by pressing the key 18.

As shown in FIG. 1, the power supply apparatus 16 includes a wireless charging receiver module 161 and a power supply module 162. The wireless charging receiver module 161 is configured to receive electric energy and charge the power supply module. The power supply module 162 is configured to supply the electric energy to the blood oxygen sensor, the display screen and the data processing module. A polymer lithium battery may be used as a battery in the power supply module, which has small volume and long power supply duration. In addition, a charging interface corresponding to the wireless charging receiver module is a reverse polarity protection interface.

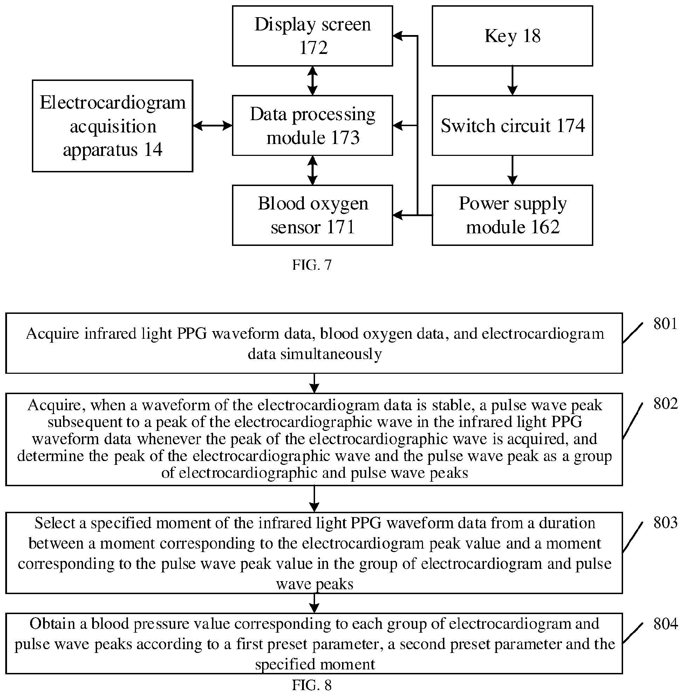

As shown in FIG. 7, when the key 18 of the measurement device in the turned-off state is pressed, a switch circuit 174 in the circuit mainboard 17 controls the power supply module 162 to supply electric energy to the blood oxygen sensor 171, the display screen 172 and the data processing module 173 after receiving a turn-on signal, so that the data processing module 173 receives the digital signal and the electrocardiogram data collected by the blood oxygen sensor 171 and the electrocardiogram collection apparatus 14, converts the digital signal into blood oxygen data, and obtains the blood pressure value by processing the infrared light PPG waveform data and the electrocardiogram data.

In order to facilitate portability of the measurement device, the measurement device may further include a through hole (not shown) provided on either side of the measurement device for tethering the measurement device. For example, the through hole is configured to tether a key chain, which is convenience to carrying the measurement device and measuring at any time.

According to the embodiment of the present disclosure, the blood oxygen measurement device and the electrocardiogram measurement device are integrated together, and the blood oxygen data and the electrocardiogram data at the same time can be simultaneously acquired by one measurement, so that the blood pressure value can be obtained by the blood oxygen data and the electrocardiogram data.

FIG. 8 is a flowchart of a measurement method of the measurement device according to the embodiments of the present disclosure, in conjunction with the measurement device in FIGs. 1 to 7 described above. As shown in FIG. 8, the method includes acts 801 to 804.

In the act 801, infrared light PPG waveform data, blood oxygen data, and electrocardiogram data are acquired simultaneously.

In the act 802, when a waveform of the electrocardiogram data is stable, a pulse wave peak subsequent to the electrocardiogram wave peak is acquired in the infrared light PPG waveform data whenever the electrocardiogram wave peak is acquired, and the electrocardiogram wave peak and the pulse wave peak are determined as a group of electrocardiogram and pulse wave peaks.

In the act 803, a specified time of the infrared light PPG waveform data is selected from a duration between the time corresponding to the electrocardiogram wave peak and the time corresponding to the pulse wave peak in the group of electrocardiogram and pulse wave peaks.

In the act 804, according to a first preset parameter, a second preset parameter and the specified time, a blood pressure value corresponding to each group of electrocardiogram and pulse wave peaks is obtained.

When left and right fingers of a user hold the first electrode slice and the second electrode slice on the measurement device shown in FIG. 2 at the same time, the infrared light PPG waveform data, the blood oxygen data and the electrocardiogram data can be obtained, simultaneously.

The specified time includes times corresponding to a maximum value, a minimum value, a first derivative, a second derivative, or a tangent point value in the infrared light PPG waveform data, from the duration between the time corresponding to the electrocardiogram peak and the time corresponding to the pulse wave peak in the group of electrocardiogram and pulse wave peaks. In addition, the first derivative is a maximum value among differences between two adjacent pieces of data in the infrared light PPG waveform data. The second derivative is a maximum value among differences between the two adjacent difference values. That is, after calculating the first derivative, the maximum value obtained by further differentiating the two adjacent difference values among all the obtained difference values is the second derivative.

Then, according to a first preset parameter, a second preset parameter and the specified time, a blood pressure value corresponding to each group of electrocardiogram and pulse wave peaks is obtained. In a process of calculating a Blood Pressure (BP) value according to the embodiment of the present disclosure, any one of the following three formulas may be used:

$$BP = \frac{a}{PAT} + b \qquad\qquad \text{formula (1)}$$

$$BP = \frac{a}{PAT^2} + b \qquad\qquad \text{formula (2)}$$

$$BP = a \cdot \ln(PAT) + b \qquad\qquad \text{formula (3)}$$

where $PAT$ is the specified time, $a$ is a first preset parameter, and b is a second preset parameter.

In addition, the blood pressure value includes a diastolic blood pressure and a systolic blood pressure, for which corresponding first preset parameter and second preset parameter are also set, respectively. That is, the first preset parameter includes a first preset parameter for the diastolic blood pressure and a first preset parameter for the systolic blood pressure, and the second preset parameter includes a second preset parameter for the diastolic blood pressure and a second preset parameter for the systolic blood pressure. In an embodiment of the present disclosure, the first derivative is optimally selected for the specified time, and the formula (3) is optimally selected for calculating the blood pressure value.

According to the embodiments of the present disclosure, the blood pressure value can be obtained when the electrocardiogram data and the infrared light PPG waveform data are obtained, and the embodiment of the present disclosure is more convenient than conventional blood pressure measurement methods in that, both a number of measurement devices and a number of measurements by the user are reduced. Compared with the conventional blood pressure measurement methods in which only one set of blood pressure values (including a diastolic blood pressure and a systolic blood pressure) can be obtained, in the embodiments of the present disclosure, a plurality of sets of blood pressure values can be obtained when the electrocardiogram data and the infrared light PPG waveform data are acquired in real time, which can monitor the blood pressure value uninterruptedly.

Moreover, a machine-readable storage medium having instructions stored thereon for causing a machine to perform the measurement method described in the embodiments described above is also provided in another aspect of the embodiments of the present disclosure.

It will be appreciated by those of skilled in the art that embodiments of the present application may be provided as methods, systems, or computer program products. Accordingly, the present application may take the form of an entirely hardware embodiment, an entirely software embodiment, or an embodiment combining software and hardware aspects. Moreover, the present application may take the form of a computer program product implemented on one or more computer-usable storage media (including, but not limited to, magnetic disk storage, CD-ROM, optical memory, etc.) containing computer-usable program code therein.

The present application is described with reference to flowcharts and/or block diagrams for the methods, devices (systems), and computer program products according to embodiments of the present application. It should be understood that each flow and/or block in the flowchart and/or block diagram, as well as combinations of the flowchart and/or block diagram, may be implemented by computer program instructions. These computer program instructions may be provided to a processor of a general purpose computer, a special purpose computer, an embedded processor, or other programmable data processing apparatus to produce a machine such that the instructions executed by the processor of the computer or other programmable data processing apparatus produce means for implementing the functions specified in one or more flows of the flowcharts and/or one or more blocks of the block diagrams.

These computer program instructions may also be stored in a computer-readable memory that can direct a computer or other programmable data processing device to operate in a specific manner, such that the instructions stored in the computer-readable memory produce an article of manufacture including instruction apparatus that implement the functions specified in one or more flows of the flowchart and/or one or more blocks of the block diagram.

These computer program instructions may also be loaded onto a computer or other programmable data processing apparatus such that a series of operational steps are performed on the computer or other programmable apparatus to produce a computer-implemented process, such that the instructions executed on the computer or other programmable apparatus provide acts for implementing the functions specified in one flow or more flows of the flowcharts and/or one block or more blocks of the block diagrams.

In one typical configuration, the computing device includes one or more processors (CPUs), input/output interfaces, network interfaces, and memory.

The memory may include non-persistent memory in a computer-readable medium, random access memory (RAM), and/or non-volatile memory, such as read-only memory (ROM) or flash RAM. The memory is an example of a computer-readable medium.

The computer-readable medium, including permanent and non-permanent, removable and non-removable media, may be stored by any method or technique to enable information storage. The information may be computer-readable instructions, data structures, modules of programs, or other data. For example, the storage medium of the computer include, but is not limited to, Phase-Change Random Access Memory (PRAM), Static Random Access Memory (SRAM), Dynamic Random Access Memory (DRAM), other types of Random Access Memory (RAM), Read-only Memory (ROM), Electrically Erasable Programmable Read-only Memory (EEPROM), flash memory or other memory technology, CD-ROM, Digital Versatile Disc (DVD) or other optical storage, magnetic cartridge, magnetic storage or other magnetic storage device, or any other non-transmission medium, may be used to store information that can be accessed by a computing device. As defined herein, computer-readable media does not include transitory media, such as modulated data signals and carrier waves.

It should also be noted that the terms "including," "containing," or any other variation thereof are intended to encompass a non-exclusive inclusion such that a process, method, commodity, or apparatus including a series of elements includes not only those elements, but also other elements not explicitly listed, or elements inherent to such a process, method, commodity, or device. An element defined by a statement "include one ..." does not exclude presence of additional identical elements in the process, method, commodity or device that includes the element, without more limitations.

The above description is only embodiments of the present application, and is not intended to limit the present application. Various modifications and variations of the present application will be apparent to those of skilled in the art. Any modification, equivalent substitution, improvement and the like made within the spirit and principle of the present application should be included within the scope of the claims of the present application.

## Claims

1. A measurement device comprising an upper housing and a lower housing, and a blood oxygen collection apparatus, an electrocardiogram collection apparatus and a display screen lens which are embedded in a groove of the upper housing, and a power supply apparatus, a circuit mainboard and a key which are provided between the upper housing and the lower housing;

   wherein the electrocardiogram collection apparatus comprises a first electrode slice and a second electrode slice that are embedded in grooves at both ends of the upper housing, respectively;
   a hollow portion is provided at a center position of any one of electrode slices, and the blood oxygen collection apparatus is disposed at the hollow portion.

2. The measurement device of claim 1, wherein through holes are provided on the upper housing and the circuit mainboard, both the first electrode slice and the second electrode slice are provided with pins, and two pins connect the two electrode slices with the circuit mainboard through the through hole of the upper housing and the through hole of the circuit mainboard.

3. The measurement device of claim 1, wherein the circuit mainboard comprises a blood oxygen sensor, a display screen and a data processing module,

   wherein the blood oxygen sensor comprises a light emitting diode and a receiving diode,
   the light emitting diode is configured to emit red light and infrared light,
   the receiving diode is configured to receive multi-channel optical signals resulting from reflecting or transmitting the red light and infrared light from human tissues, convert the multi-channel optical signals into a digital signal and transmit the digital signal to the data processing module, wherein the data signal comprises red light Photoplethysmography (PPG) waveform data and infrared light PPG waveform data;
   the data processing module is configured to receive the digital signal and electrocardiogram data, convert the digital signal into blood oxygen data, and obtain a blood pressure value by processing the infrared light PPG waveform data and the electrocardiogram data; and
   the display screen is configured to display the blood oxygen data, the electrocardiogram data and the blood pressure value through the display screen lens in the groove of the upper housing.

4. The measurement device of claim 3, wherein a first aperture, a baffle and a second aperture are provided side by side in the groove of the upper housing directly below the hollow portion,

   wherein the first aperture is configured to transmit the red light and infrared light emitted by the light emitting diode to the blood oxygen collection apparatus;
   the second aperture is configured to transmit the multi-channel optical signals to the receiving diode; and
   the baffle is configured to isolate light emitted by the light emitting diode from the multi-channel optical signals received by the receiving diode.

5. The measurement device of claim 4, wherein the blood oxygen collection apparatus comprises a light emitting lens, a reflecting lens and a light shielding pad, wherein the light shielding pad is provided with a third aperture and a fourth aperture, the third aperture is disposed above the first aperture, and the fourth aperture is disposed above the second aperture;

the light emitting lens is disposed above the third aperture, and is configured to transmit the red light and infrared light emitted by the light emitting diode, and filter out other light other than the red light and infrared light; the reflecting lens is disposed above the fourth aperture, and is configured to transmit the multi-channel optical signals and filter out other optical signals other than the multi-channel optical signals; and the light shielding pad is configured to block an influence of external light on transmission of the red light, the infrared light and the multi-channel optical signals.

6. The measurement device of claim 1, wherein the two electrode slices are made of gold plated copper or nickel plated copper.

7. The measurement device of claim 1, wherein the key is configured to start or stop acquiring electrocardiogram data and blood oxygen data of a user, when pressed once in control.

8. The measurement device of claim 1, wherein the measurement device further comprises a through hole provided on either side of the measurement device for tethering the measurement device.

9. The measurement device of claim 3, wherein the power supply apparatus comprises a wireless charging receiver module and a power supply module,

the wireless charging receiver module is configured to receive electric energy and charge the power supply module; and the power supply module is configured to supply the electric energy to the blood oxygen sensor, the display screen and the data processing module.

10. The measurement device of claim 3, wherein the data processing module comprises an Analog Front End (AFE) chip, a first Micro Control Unit (MCU), a serial interface, a second MCU, a data storage module, a clock module and a communication module;

the AFE chip is configured to simultaneously collect the digital signal and the electrocardiogram data, and convert the digital signal into the blood oxygen data; the first MCU is configured to process the infrared light PPG waveform data and the electrocardiogram data to obtain the blood pressure value; the serial interface is configured to transfer the blood oxygen data, the electrocardiogram data and the blood pressure value from the first MCU to the second MCU; the second MCU is configured to receive the blood oxygen data, the electrocardiogram data and the blood pressure value, and transmit the blood oxygen data, the electrocardiogram data and the blood pressure value to the data storage module, the display screen and the communication module; the data storage module is configured to store the blood oxygen data, the electrocardiogram data and the blood pressure value; the communication module is configured to transmit the blood oxygen data, the electrocardiogram data and the blood pressure value to a communication terminal; and the clock module is configured to provide time.

11. The measurement device of claim 10, wherein obtaining the blood pressure value by processing the infrared light PPG waveform data and the electrocardiogram data comprises:

acquiring, when a waveform of the electrocardiogram data is stable, a pulse wave peak subsequent to an electrocardiogram wave peak in the infrared light PPG waveform data whenever the electrocardiogram wave peak is acquired, and determining the electrocardiogram wave peak and the pulse wave peak as a group of electrocardiogram and pulse wave peaks; selecting a specified time of the infrared light PPG waveform data from a duration between time corresponding to the electrocardiogram wave peak and time corresponding to the pulse wave peak in the group of electrocardiogram and pulse wave peaks; and
obtaining a blood pressure value corresponding to each group of electrocardiogram and pulse wave peaks according to a first preset parameter, a second preset parameter and the specified time.

12. The measurement device of claim 11, wherein the specified time comprises times corresponding to a maximum value, a minimum value, a first derivative, a second derivative, or a tangent point value in the infrared light PPG waveform data, from the duration between the time corresponding to the electrocardiogram wave peak and the time corresponding to the pulse wave peak in the group of electrocardiogram and pulse wave peaks.

13. The measurement device of claim 12, wherein the first derivative is a maximum value among differences between two adjacent pieces of data in the infrared light PPG waveform data, and the second derivative is a maximum value among differences between two adjacent differences.

14. The measurement device of claim 11, wherein obtaining the blood pressure value corresponding to each group of electrocardiogram and pulse wave peaks according to the first preset parameter, the second preset parameter and the specified time comprises:

according to

$$\mathrm{BP} = \frac{a}{PAT} + b,$$

or

$$\mathrm{BP} = \frac{a}{PAT^2} + b,$$

or

$$\mathrm{BP} = a \cdot \ln(PAT) + b,$$

obtaining the Blood Pressure (BP) value, wherein $PAT$ is the specified time, $a$ is the first preset parameter, and b is the second preset parameter.

15. The measurement device of claim 14, wherein the blood pressure value comprises a diastolic blood pressure and a systolic blood pressure, the first preset parameter comprises a first preset parameter for the diastolic blood pressure and a first preset parameter for the systolic blood pressure, and the second preset parameter comprises a second preset parameter for the diastolic blood pressure and a second preset parameter for the systolic blood pressure.

16. A measurement method of the measurement device of any one of claims 1 to 15, comprising:

acquiring infrared light Photoplethysmography (PPG) waveform data, blood oxygen data and electrocardiogram data at the same time;
acquiring, when a waveform of the electrocardiogram data is stable, a pulse wave peak subsequent to an electrocardiogram wave peak in the infrared light PPG waveform data whenever the electrocardiogram wave peak is acquired, and determining the electrocardiogram wave peak and the pulse wave peak as a group of electrocardiogram and pulse wave peaks;
selecting a specified time of the infrared light PPG waveform data from a duration between time corresponding to the electrocardiogram wave peak and time corresponding to the pulse wave peak in the group of electrocardiogram and pulse wave peaks; and
obtaining a blood pressure value corresponding to each group of electrocardiogram and pulse wave peaks according to a first preset parameter, a second preset parameter and the specified time.

17. The measurement method of claim 16, wherein the specified time comprises times corresponding to a maximum value, a minimum value, a first derivative, a second derivative, or a tangent point value in the infrared light PPG waveform data, from the duration between the time corresponding to the electrocardiogram wave peak and the time corresponding to the pulse wave peak in the group of electrocardiogram and pulse wave peaks.

18. The measurement method of claim 17, wherein the first derivative is a maximum value among differences between two adjacent pieces of data in the infrared light PPG waveform data, and the second derivative is a maximum value among differences between two adjacent differences.

19. The measurement method of claim 16, wherein obtaining the blood pressure value corresponding to each group of electrocardiogram and pulse wave peaks according to the first preset parameter, the second preset parameter and the specified time comprises:

according to

$$BP = \frac{a}{PAT} + b,$$

or

$$BP = \frac{a}{PAT^2} + b,$$

or

$$BP = a \cdot \ln(PAT) + b,$$

obtaining the Blood Pressure (BP) value, wherein *PAT* is the specified time, *a* is the first preset parameter, and b is the second preset parameter.

20. The measurement method of claim 19, wherein the blood pressure value comprises a diastolic blood pressure and a systolic blood pressure, the first preset parameter comprises a first preset parameter for the diastolic blood pressure and a first preset parameter for the systolic blood pressure, and the second preset parameter comprises a second preset parameter for the diastolic blood pressure and a second preset parameter for the systolic blood pressure.

21. A machine-readable storage medium having stored thereon instructions for causing a machine to perform the measurement method of any one of claims 16 to 20.

100

13

143

15

141

142

14

11

17

18

161

16

162

12

FIG. 1

FIG. 2

FIG. 3

FIG. 4

FIG. 5

| AFE chip 51 | First MCU 52 |
|---|---|

Serial interface 53

| Data storage module 55 | Second MCU 54 | Communication module 57 |
|---|---|---|

Clock module 56

FIG. 6

```
┌─────────────────┐          ┌─────────────────┐
│  Display screen │◄──┐      │     Key 18      │
│       172       │   │      │                 │
└─────────────────┘   │      └─────────────────┘
         ▲▼           │               │
┌──────────────┐ ┌─────────────────┐  ▼       ┌─────────────────┐
│Electrocardio-│ │ Data processing │◄─┤       │Switch circuit 174│
│    gram      │◄►│   module 173   │          └─────────────────┘
│ acquisition  │ └─────────────────┘               │
│ apparatus 14 │         ▲▼                         ▼
└──────────────┘ ┌─────────────────┐      ┌─────────────────┐
                 │  Blood oxygen   │◄─────│  Power supply   │
                 │   sensor 171    │      │  module 162     │
                 └─────────────────┘      └─────────────────┘
```

FIG. 7

Acquire infrared light PPG waveform data, blood oxygen data, and electrocardiogram data simultaneously ⟋ 801

Acquire, when a waveform of the electrocardiogram data is stable, a pulse wave peak subsequent to a peak of the electrocardiographic wave in the infrared light PPG waveform data whenever the peak of the electrocardiographic wave is acquired, and determine the peak of the electrocardiographic wave and the pulse wave peak as a group of electrocardiographic and pulse wave peaks ⟋ 802

Select a specified moment of the infrared light PPG waveform data from a duration between a moment corresponding to the electrocardiogram peak value and a moment corresponding to the pulse wave peak value in the group of electrocardiogram and pulse wave peaks ⟋ 803

Obtain a blood pressure value corresponding to each group of electrocardiogram and pulse wave peaks according to a first preset parameter, a second preset parameter and the specified moment ⟋ 804

FIG. 8

## INTERNATIONAL SEARCH REPORT

International application No.

**PCT/CN2023/109470**

| A. | CLASSIFICATION OF SUBJECT MATTER |
|---|---|

A61B5/1455(2006.01)i;  A61B5/0225(2006.01)i

According to International Patent Classification (IPC) or to both national classification and IPC

| B. | FIELDS SEARCHED |
|---|---|

Minimum documentation searched (classification system followed by classification symbols)

IPC:A61B

Documentation searched other than minimum documentation to the extent that such documents are included in the fields searched

Electronic data base consulted during the international search (name of data base and, where practicable, search terms used)

CNTXT, ENTXTC, WPABSC, ENTCT, DWPI: 电极, 洞, 间隙, 空隙, 孔, 心电, 血压, 血氧, 镂空, electrode+, hole+, gap, pore, electrocardiogram, blood, pressure, oxygen, hollow+, PPG

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115211851 A (BEIJING CHOICE ELECTRONIC TECHNOLOGY CO., LTD.) 21 October 2022 (2022-10-21)<br>claims 1-15 and 21 | 1-15, 21 |
| X | CN 210383886 U (SHENZHEN ICARBONX INTELLIGENT DIGITAL LIFE HEALTH MANAGEMENT CO., LTD.) 24 April 2020 (2020-04-24)<br>description, paragraphs 57-120, and figures 1-18 | 1-10 |
| Y | CN 210383886 U (SHENZHEN ICARBONX INTELLIGENT DIGITAL LIFE HEALTH MANAGEMENT CO., LTD.) 24 April 2020 (2020-04-24)<br>description, paragraphs 57-120, and figures 1-18 | 11-15, 21 |
| X | CN 107438402 A (NOKIA TECHNOLOGIES OY) 05 December 2017 (2017-12-05)<br>description, paragraphs 34-70, and figures 1-12 | 1-10 |
| Y | US 2014031646 A1 (YAKIREVICH SERGEY; TAL YAIR; TAL BENNY; PRESSMAN ASSAF;) 30 January 2014 (2014-01-30)<br>description, paragraphs 18-178, and figures 1-17 | 11-15, 21 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **08 November 2023** | **08 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/109470** |

**C.      DOCUMENTS CONSIDERED TO BE RELEVANT**

| Category* | Citation of document, with indication, where appropriate, of the relevant passages | Relevant to claim No. |
| --- | --- | --- |
| A | CN 102293640 A (QINHUANGDAO CONTEC MEDICAL SYSTEMS CO., LTD.) 28 December 2011 (2011-12-28)<br>entire document | 1-15, 21 |
| A | US 6470885 B1 (BLUE-I) BLUE B; (STOK-I) STOKES S29 October 2002 (2002-10-29)<br>entire document | 1-15, 21 |

Form PCT/ISA/210 (second sheet) (July 2022)

## INTERNATIONAL SEARCH REPORT

| International application No. |
| --- |
| **PCT/CN2023/109470** |

**Box No. II**     **Observations where certain claims were found unsearchable (Continuation of item 2 of first sheet)**

This international search report has not been established in respect of certain claims under Article 17(2)(a) for the following reasons:

1. ☑ Claims Nos.: **16-20**
   because they relate to subject matter not required to be searched by this Authority, namely:

   Claims 16-20 relate to a method for measuring blood pressure, which falls within methods for measuring body blood pressure, is a diagnosis method implemented on a living human or animal body, and falls within subject matter to be excluded (PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

INTERNATIONAL SEARCH REPORT
Information on patent family members

International application No.

**PCT/CN2023/109470**

| Patent document cited in search report | | | Publication date (day/month/year) | Patent family member(s) | | | Publication date (day/month/year) |
|---|---|---|---|---|---|---|---|
| CN | 115211851 | A | 21 October 2022 | None | | | |
| CN | 210383886 | U | 24 April 2020 | WO | 2020147559 | A1 | 23 July 2020 |
| CN | 107438402 | A | 05 December 2017 | WO | 2016166414 | A1 | 20 October 2016 |
| | | | | EP | 3081152 | A1 | 19 October 2016 |
| | | | | EP | 3081152 | B1 | 07 December 2022 |
| | | | | US | 2018020975 | A1 | 25 January 2018 |
| | | | | US | 11272882 | B2 | 15 March 2022 |
| US | 2014031646 | A1 | 30 January 2014 | WO | 2013144968 | A1 | 03 October 2013 |
| | | | | EP | 2644089 | A1 | 02 October 2013 |
| | | | | EP | 2644089 | B1 | 28 December 2016 |
| | | | | EP | 3205263 | A1 | 16 August 2017 |
| | | | | EP | 3205263 | B1 | 19 June 2019 |
| | | | | DK | 3205263 | T3 | 19 August 2019 |
| | | | | DK | 2644089 | T3 | 20 March 2017 |
| CN | 102293640 | A | 28 December 2011 | None | | | |
| US | 6470885 | B1 | 29 October 2002 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210892732